# EUROPEAN PATENT APPLICATION

(11) **EP 2 682 752 A1**
(43) Date of publication of application: **08.01.2014**
(21) Application number: 12175017.8
(22) Date of filing: 04.07.2012
(51) Int. Cl.: G01N 33/68

(54) **HMGA2 as a marker for diagnosing diabetes**

(71) Applicant: alcedo biotech GmbH, 28359 Bremen (DE)
(72) Inventor: Bullerdiek, Jörn, 28359 Bremen (DE)
(74) Representative: Fanelli Haag Kilger PLLC

(57) **Abstract**

The invention relates to a method for diagnosis and/or prognosis of diabetes, comprising the steps of a) analyzing in a sample of a subject the expression level or status of the gene HMGA2 wherein, b) if HMGA2 shows an elevated expression equal to or above a cut-off value when compared to a control, c) the subject is classified as a subject with diabetes or the subject is classified as a subject with poor prognosis or the subject is classified as a subject being at an elevated risk of becoming diabetic.

## Description

### FIELD OF THE INVENTION

The present invention is in the field of biology, medicine and chemistry. In particular, the invention is in the field of molecular biology and diagnostics. More particular, the invention relates to the analysis of diagnosis and prognosis of diabetes.

### BACKGROUND

Type 2 diabetes mellitus is characterized by insulin resistance, which may be combined with relatively reduced insulin secretion. The defective responsiveness of body tissues to insulin is believed to involve the insulin receptor. However, the specific defects are not known. Diabetes mellitus cases due to a known defect are classified separately. Type 2 diabetes is the most common type.

In the early stage of type 2, the predominant abnormality is reduced insulin sensitivity. At this stage, hyperglycemia can be reversed by a variety of measures and medications that improve insulin sensitivity or reduce glucose production by the liver.

Type 2 diabetes and the burden of diseases related to it are rapidly increasing worldwide. Thus, the term of a diabetic epidemic has been coined. Accordingly, the socioeconomic consequences of this epidemic are enormous and still rising. While most likely causes as, in particular, obesity, poor diet, and ageing of populations have been identified, an as yet challenging problem is to identify individuals who are at risk of getting the disease. There is no serious doubt that a larger portion of individuals with diabetes are under diagnosed but are at risk of getting serious problems related to the disease.

Also for diabetes there is need for additional reliable diagnostic and prognostic markers.

### DEFINITIONS

The following definitions are provided for specific terms which are used in the following.

As used herein, the term "composition" refers to any mixture. It can be a solution, a suspension, liquid, powder, a paste, aqueous, non-aqueous or any combination thereof.

As used herein, the term "diagnosis" refers to the identification of a subject with diabetes.

The term "prognosis" as used herein refers to a prediction of the probable course and outcome of a clinical condition or disease. A prognosis of a patient is usually made by evaluating factors, markers, and/or symptoms of a disease that are indicative of a favourable or unfavourable course or outcome of the disease. Preferably herein prognosis involves determining the probability that an early diabetes will develop into a late stage diabetes. In the early stage of type 2, the predominant abnormality is reduced insulin sensitivity. At this stage, hyperglycemia can be reversed by a variety of measures and medications that improve insulin sensitivity or reduce glucose production by the liver. Hence, prognosis herein means assessing the probability of progression into a late stage diabetes, and/or the development of an early stage diabetes.

The phrase "determining the prognosis" as used herein refers to the process by which the skilled artisan can predict the course or outcome of a condition in a patient. The term "prognosis" does not refer to the ability to predict the course or outcome of a condition with 100 % accuracy. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition. A prognosis may be expressed as the amount of time a patient can be expected to survive. Alternatively, a prognosis may refer to the likelihood that the disease goes into remission or to the amount of time the disease can be expected to remain in remission. Prognosis can be expressed in various ways; for example prognosis can be expressed as a percent chance that a patient will survive after one year, five years, ten years or the like. Alternatively, prognosis may be expressed as the number of years, on average, that a patient can expect to survive as a result of a condition or disease. The prognosis of a patient may be considered as an expression of relativism, with many factors effecting the ultimate outcome. For example, for patients with certain conditions, prognosis can be appropriately expressed as the likelihood that a condition may be treatable or curable, or the likelihood that a disease will go into remission, whereas for patients with more severe conditions prognosis may be more appropriately expressed as likelihood of survival for a specified period of time.

Herein, a "cut-off value" is defined as follows: a specific expression level above which results are regarded as positive (or negative for a gene with a reverse association) versus when the expression level is below the cut-off the results are regarded as negative (or positive for a gene with reverse association). To account for biological variability that is known to be typical of all living biological systems such as humans or other organisms it is reasonable to consider ranges of values and thus all cut-off values herein may vary by plus minus 15 %, plus minus 10 % or preferably only plus minus 5 %. This also depends on the experimental set-up.

The term "analyzing the expression status or level" as used herein, relates to the means and methods useful for assessing the expression status. Useful methods are all quantitative molecular methods, such as transcript sequencing, real-time amplification or PCR, hybridization, or the like.

As used herein, "isolated" when used in reference to a nucleic acid means that a naturally occurring sequence has been removed from its normal cellular (e.g. chromosomal) environment or is synthesised in a non-natural environment (e.g. artificially synthesised). Thus, an "isolated" sequence may be in a cell-free solution or placed in a different cellular environment.

As used herein, a "kit" is a packaged combination optionally including instructions for use of the combination and/or other reactions and components for such use.

As used herein, "nucleic acid(s)" or "nucleic acid molecule" generally refers to any ribonucleic acid or deoxyribonucleic acid, which may be unmodified or modified DNA or RNA. "Nucleic acids" include, without limitation, single- and double-stranded nucleic acids. As used herein, the term "nucleic acid(s)" also includes DNA, as described above that contain one or more modified bases. Thus, DNA with backbones modified for stability or for other reasons are "nucleic acids". The term "nucleic acid(s)" as it is used herein embraces such chemically, enzymatically or metabolically modified forms of nucleic acids, as well as the chemical forms of DNA characteristic of viruses and cells, including for example, simple and complex cells.

The term "primer" as used herein refers to a nucleic acid, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and the method used. For example, for diagnostic applications, depending on the complexity of the target sequence, the nucleic acid primer typically contains 15-25 or more nucleotides, although it may contain fewer nucleotides.

As used herein, the term "probe" means nucleic acid and analogs thereof and refers to a range of chemical species that recognise polynucleotide target sequences through hydrogen bonding interactions with the nucleotide bases of the target sequences. The probe or the target sequences may be single- or double-stranded DNA. A probe is at least 8 nucleotides in length and less than the length of a complete polynucleotide target sequence. A probe may be 10, 20, 30, 50, 75, 100, 150, 200, 250, 400, 500 and up to 10,000 nucleotides in length. Probes can include nucleic acids modified so as to have one or more tags which are detectable by fluorescence, chemiluminescence and the like ("labelled probe"). The labelled probe can also be modified so as to have both one or more detectable tags and one or more quencher molecules, for example Taqman® and Molecular Beacon® probes. The nucleic acid and analogs thereof may be DNA, or analogs of DNA, commonly referred to as antisense oligomers or antisense nucleic acid. Such DNA analogs comprise but are not limited to 2-'O-alkyl sugar modifications, methylphosphonate, phosphorothiate, phosphorodithioate, formacetal, 3'-thioformacetal, sulfone, sulfamate, and nitroxide backbone modifications, and analogs wherein the base moieties have been modified. In addition, analogs of oligomers may be polymers in which the sugar moiety has been modified or replaced by another suitable moiety, resulting in polymers which include, but are not limited to, morpholino analogs and peptide nucleic acid (PNA) analogs (Egholm, et al. Peptide Nucleic Acids (PNA)-Oligonucleotide Analogues with an Achiral Peptide Backbone, (1992)).

The term "sample" is used herein to refer to tissue per se or tissue such as white adipose tissue (WAT). The term "sample" or "biological sample" as used herein also refers to tissue, blood, urine, semen, secretions or isolated cells originating from a subject, preferably from WAT tissue. Most preferably the term sample means human subcutaneous abdominal adipose white tissue (hWAT).

As used herein, "stringent conditions for hybridization" are known to those skilled in the art and can be found in Current Protocols in Molecular Biology, John Wiley & Sons, N.Y., 6.3.1-6.3.6, 1991. Stringent conditions are defined as equivalent to hybridization in 6X sodium chloride/sodium citrate (SSC) at 45 °C, followed by a wash in 0.2 X SSC, 0.1 % SDS at 65 °C.

As used herein, the terms "subject" and "patient" are used interchangeably to refer to a human or an animal (e.g., a mammal, a fish, an amphibian, a reptile, a bird or an insect). In a specific embodiment, a subject is a mammal (e.g., a non-human mammal or a human). In another embodiment, a subject is a pet (e.g., a dog, a cat, a guinea pig, a monkey or a bird), a farm animal (e.g., a horse, a cow, a pig, a goat or a chicken) or a laboratory animal (e.g., a mouse or a rat). In another embodiment, a subject is a primate (e.g., a chimpanzee or a human). In another embodiment, a subject is a human. In another embodiment, the subject is a male human or a female human.

### DETAILED DESCRIPTION OF THE INVENTION

The invention as disclosed herein identifies genomic regions that are useful in diagnosing diabetes. These are also prognostic markers.

By definition, the identified genomic regions are biomarkers for diabetes. In order to use these genomic regions (as biomarkers), the invention teaches the analysis of the expression status or level of said genomic regions. The invention further encompasses genomic region specific nucleic acids. The invention further contemplates the use of said genomic region specific nucleic acids to analyze the expression level, either directly or indirectly by methods known to the skilled person and explained herein. The invention further discloses a composition and kit comprising said nucleic acids for the diagnosis of diabetes.

The inventors found genomic regions that are subject to an aberrant expression level in subjects with diabetes.

Hence, the invention relates to a method for diagnosis and/or prognosis of diabetes, comprising the steps of
a. analyzing in a sample of a subject the expression level or status of the gene HMGA2 according to SEQ ID NO. 1 wherein,
b. if HMGA2 shows an elevated expression equal to or above a cut-off value when compared to a control,
c. the subject is classified as a subject with diabetes or the subject is classified as a subject with poor prognosis.

HMGA2 is the gene encoding the high mobility group AT-hook 2 protein (HMGA2). This gene encodes a protein that belongs to the non-histone chromosomal high-mobility group (HMG) protein family. HMG proteins function as architectural factors. This protein contains structural DNA-binding domains and may act as a transcriptional regulating factor. Identification of the deletion, amplification, and rearrangement of this gene that are associated with lipomas suggested a role in adipogenesis and mesenchymal differentiation. This is one reason why the present finding is so astonishing.

A gene knock-out study of the mouse counterpart demonstrated that this gene is involved in diet-induced obesity. Alternate transcriptional splice variants, encoding different isoforms, have been characterized. The findings herein however may contradict the above.

The expression of HMGA2 in adult tissues is commonly associated with both malignant and benign tumor formation, as well as certain characteristic cancer-promoting mutations. Homologous proteins with highly conserved sequences are found in other mammalian species, including lab mice (Mus musculus).

HMGA2 contains three basic DNA-binding domains (AT-hooks) that cause the protein to bind to adenine-thymine (AT)-rich regions of nuclear DNA. HMGA2 does not directly promote or inhibit the transcription of any genes, but alters the structure of DNA and promotes the assembly of protein complexes that do regulate the transcription of genes.

With few exceptions, HMGA2 is expressed in humans only during early development, and is reduced to undetectable or nearly undetectable levels of transcription in adult tissues.

The microRNA let-7 is largely responsible for this time-dependent regulation of HMGA2.

The apparent function of HMGA2 in proliferation and differentiation of cells during development is supported by the observation that mice with mutant HMGA2 genes are unusually small (pygmy phenotype), and genome-wide association studies linking HMGA2-associated SNPs to variation in human height.

Heightened expression of HMGA2 is found in a variety of human cancers, but the precise mechanism by which HMGA2 contributes to the formation of cancer is unknown.

The same mutations that lead to pituitary adenomas in mice can be found in similar cancers in humans. Its presence is associated with poor prognosis for the patient, but also with sensitization of the cancer cells to certain forms of cancer therapy.

To be specific, HMGA2-high cancers display an abnormally strong response to double-strand breaks in DNA caused by radiation therapy and some forms of chemotherapy. Artificial addition of HMGA2 to some forms of cancer unresponsive to DNA damage cause them to respond to the treatment instead, although the mechanism by which this phenomenon occurs is also not understood.

However, the expression of HMGA2 is also associated with increased rates of metastasis in breast cancer, and both metastasis and recurrence of squamous cell carcinoma. These properties are responsible for patients' poor prognoses. As with HMGA2's effects on the response to radiation and chemotherapy, the mechanism by which HMGA2 exerts these effects is unknown.

To date no relationship between HMGA2 expression and diabetes has been shown.

The level of gene expression in the present method may be determined with a nucleic acid based method or by determining whether the translated protein is present and how much of it is present in the sample. A nucleic acid based method is however preferred.

Preferably, the diabetes is a type 2 diabetes.

Preferably the subject is a human and said human is adipose.

In order to determine the expression level of a gene it is necessary to compare the expression level to a control. Preferably, the control is selected from the group of housekeeping genes as e.g. *HPRT, 18S, GAPDH, GUSB, PBGD, B2M, ABL, RPLP0.*

Classification of the subject is done by determining whether or not the level of expression is over or under a certain cut-off level when compared to a control. Elevated expression or said cut-off value is defined as 1.5 times elevated when compared to the control.

Preferably the sample is white adipose tissue (WAT) but other possible samples are, e.g. blood, serum, plasma.

Preferably, the expression level is determined by quantitative reverse transcriptase PCR (qRT-PCR) as e.g. a Real-Time PCR reaction or by other possible methods include array hybridization, mass spectrometry (MALDI-TOF), serial analysis of gene expression (SAGE) and other sensitive method to detect mRNAs or proteins or fragments thereof.

The method makes use of one or more nucleic acid molecules, that hybridize to the expression product of the HMGA2 gene. Hence, the invention also relates to the use of a nucleic acid molecule that hybridizes under stringent conditions to the mRNA transcript of the HMGA2 gene according to SEQ ID NO. 1 for determining the expression level of said gene in a method according to any of the invention.

Preferably, the nucleic acid is 15 to 100 nt in length.

Preferably, the nucleic acid is a primer or probe and optionally the primer or probe is labelled.

The invention also relates to the use of a kit for the diagnosis of diabetes comprising a nucleic acid that that hybridizes under stringent conditions to the mRNA transcript of the HMGA2 gene according to SEQ ID NO. 1.

Preferably, the kit is Real-Time qRT-PCR kit, or a kit suited for array hybridization, mass spectrometry (MALDI-TOF), serial analysis of gene expression (SAGE) and other sensitive method to detect mRNAs or proteins or fragments thereof.

The profile expression data may be prepared in a format suitable for interpretation by a treating clinician. For example, rather than providing raw expression data, the prepared format may represent a diagnosis or risk assessment (e.g. likelihood of diabetes being present) for the subject, along with recommendations for particular treatment options.

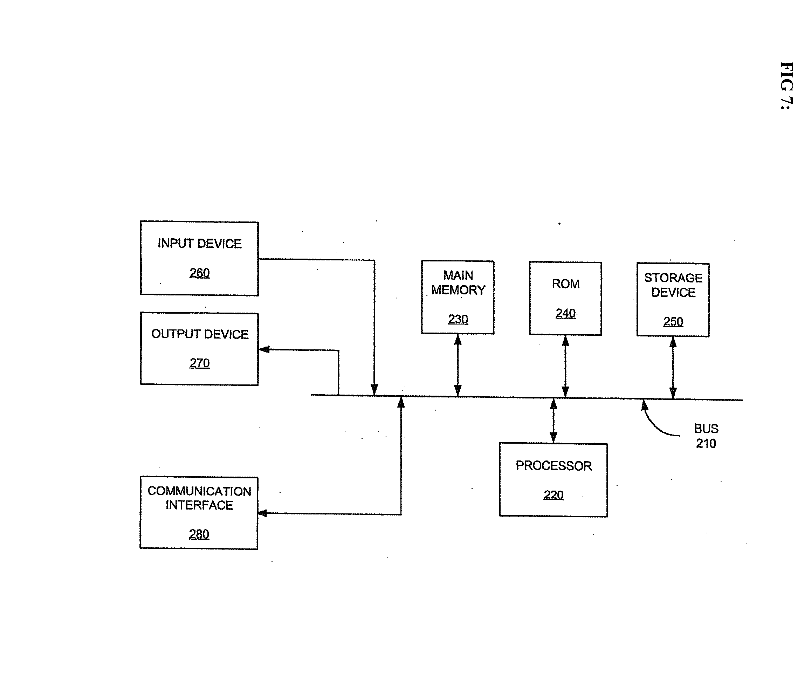

In one embodiment of the present invention, a computing device comprising a client or server component may be utilized. Figure 7 is an exemplary diagram of a client/server component, which may include a bus **210,** a processor **220,** a main memory **230,** a read only memory (ROM) **240,** a storage device **250,** an input device **260,** an output device **270,** and a communication interface **280.** Bus 210 may include a path that permits communication among the elements of the client/server component.

Processor **220** may include a conventional processor or microprocessor, or another type of processing logic that interprets and executes instructions. Main memory **230** may include a random access memory (RAM) or another type of dynamic storage device that stores information and instructions for execution by processor 220. ROM **240** may include a conventional ROM device or another type of static storage device that stores static information and instructions for use by processor **220.** Storage device **250** may include a magnetic and/or optical recording medium and its corresponding drive.

Input device 260 may include a conventional mechanism that permits an operator to input information to the client/server component, such as a keyboard, a mouse, a pen, voice recognition and/or biometric mechanisms, etc. Output device **270** may include a conventional mechanism that outputs information to the operator, including a display, a printer, a speaker, etc. Communication interface **280** may include any transceiver-like mechanism that enables the client/server component to communicate with other devices and/or systems. For example, communication interface 280 may include mechanisms for communicating with another device or system via a network.

As will be described in detail below, the client/server component, consistent with the principles of the invention, may perform certain measurement determinations of expression level, calculations of expression status, and/or correlation operations relating to the diagnosis of diabetes. It may further optionally output the presentation of status results as a result of the processing operations conducted. The client/server component may perform these operations in response to processor **220** executing software instructions contained in a computer-readable medium, such as memory **230.** A computer-readable medium may be defined as a physical or logical memory device and/or carrier wave.

The software instructions may be read into memory **230** from another computer-readable medium, such as data storage device **250,** or from another device via communication interface **280.** The software instructions contained in memory **230** may cause processor **220** to perform processes that will be described later. Alternatively, hardwired circuitry may be used in place of or in combination with software instructions to implement processes consistent with the principles of the invention. Thus, implementations consistent with the principles of the invention are not limited to any specific combination of hardware circuitry and software.

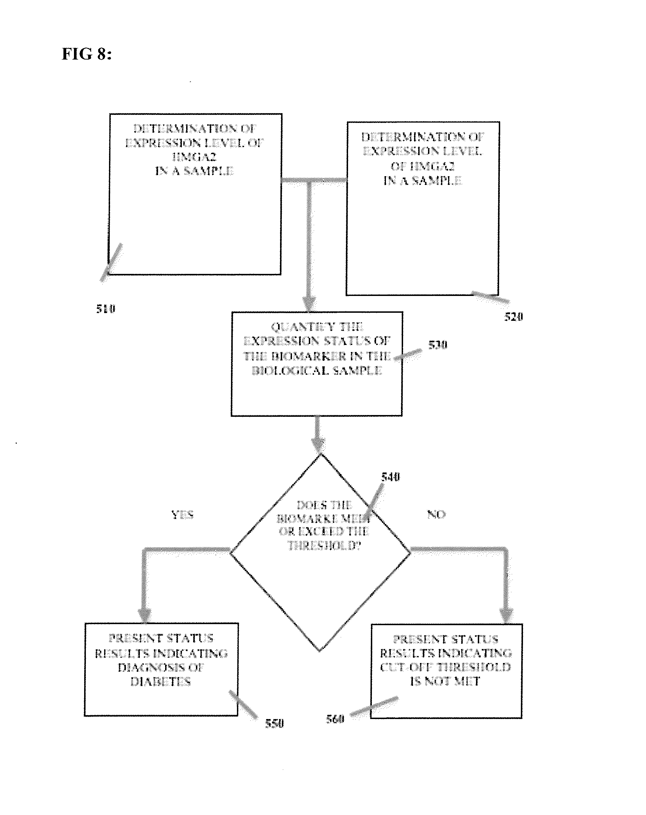

FIG. 8 is a flowchart of exemplary processing of expression status for the biomarker present in biological samples according to an implementation consistent with the principles of the present invention. Processing may begin with quantifying the expression level **510** and control **520** of the biological sample for HMGA2. The processor may then quantify the status **530,** as described above, as the ratio of HMGA2 expression to control expression of the biological sample. The expression status may then be evaluated either via a computing device **540** or by human analysis to determine if the biomarker meets or exceeds a predetermined cut-off threshold. If the threshold is met or exceeded, the computing device may then, optionally, present a status result indicating a positive diagnosis of diabetes **550.** Alternatively, if the threshold is not met, then the computing device may, optionally, present a status result indicating that the threshold is not satisfied **560.** It is noted that the output displaying results may differ depending on the desired presentation of results. For example, the output may be quantitative in nature, e.g., displaying the measurement values of each of the samples in relation to the predetermined expression threshold value. The output may be qualitative, e.g., the display of a color or notation indicating a positive result for diabetes, or a negative result for diabetes, as the case may be.

In some embodiments, the results are used in a clinical setting to determine a further diagnostic (e.g., additional further screening (e.g., other markers or diagnostic biopsy) course of action. In other embodiments, the results are used to determine a treatment course of action (e.g., choice of therapies or watchful waiting).

### FIGURE CAPTIONS

**Fig. 1****:**

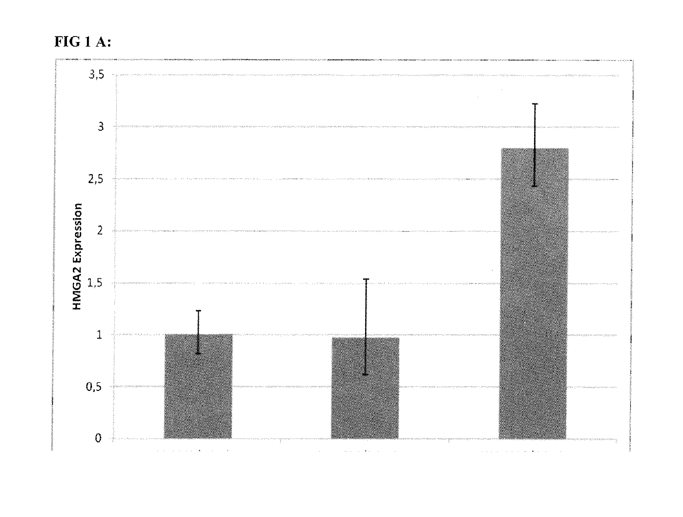

   Fig.1A: Relative HMGA2 mRNA expression in aspirates taken from three hWAT samples.

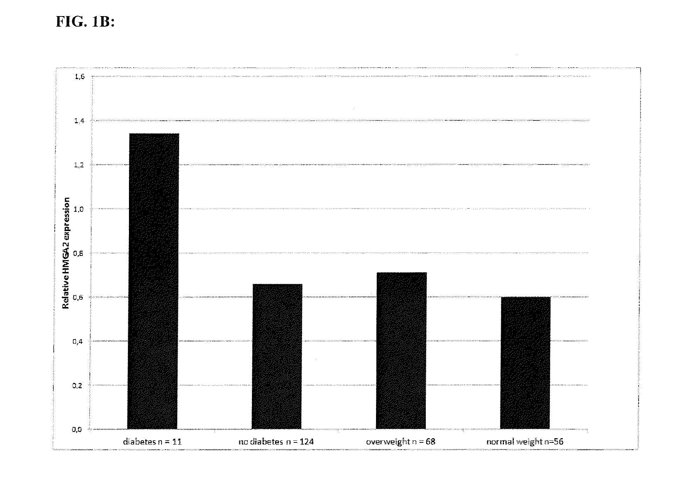

   Fig. 1B: Average expression of HMGA2 mRNA in abdominal subcutaneous adipose tissue as measured in a diabetes (n=11), no diabetes (n=124), no diabetes but overweight (n=68), and normal weight - no diabetes group. The complete study group included 135 individuals.
**Fig. 2****:**

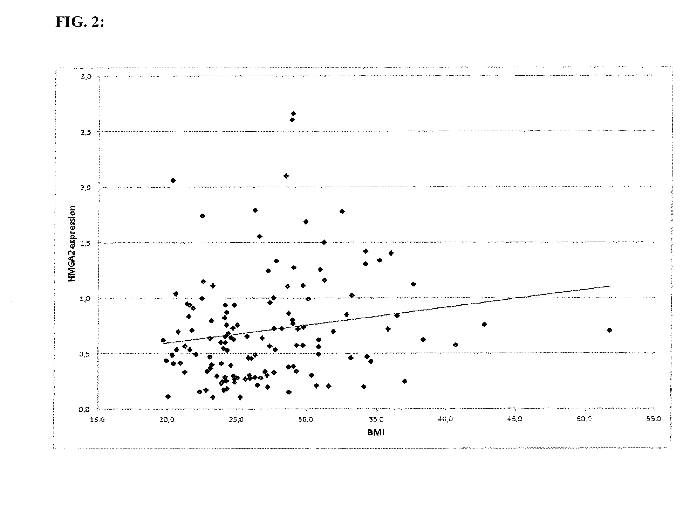

   Fig. 2: HMGA2 expression does not correlate with BMI. HMGA2 expression in hWAT plotted against the BMI of 134 individuals.
**Fig. 3****:**

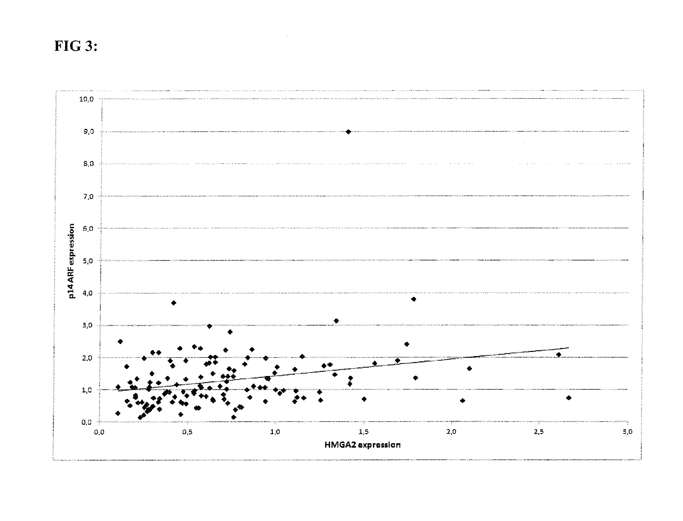

   Fig. 3: p14Arf and HMGA2 mRNA expression in hWAT revealing a significant correlation.
**Fig. 4****:**

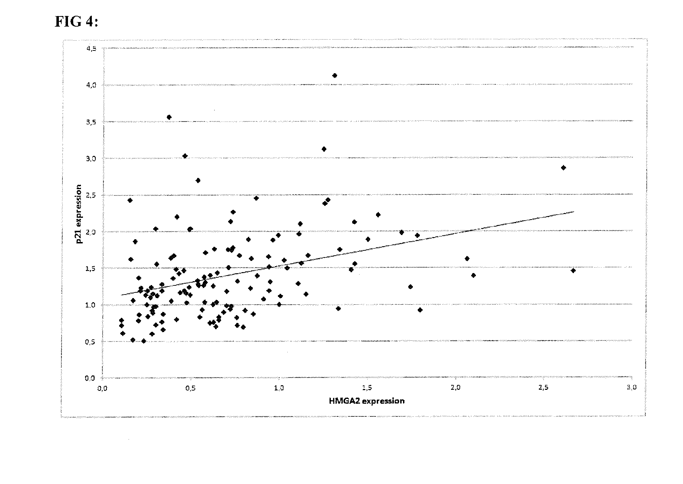

   Fig. 4: PPARg and HMGA2 mRNA expression in hWAT revealing an inverse correlation.
**Fig. 5****:**

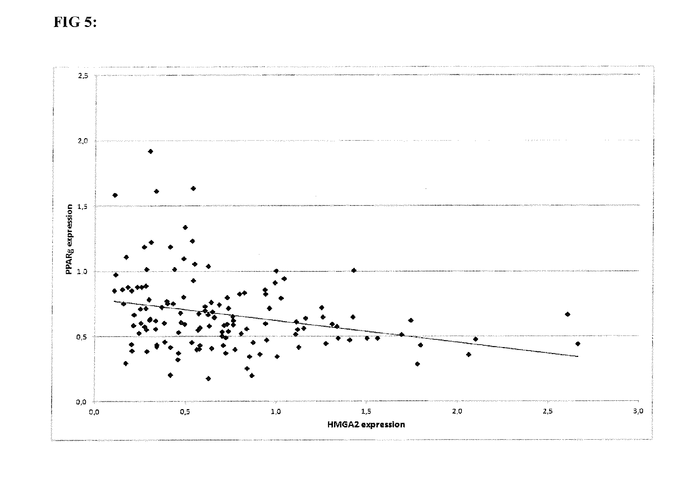

   Fig. 5: PPARgamma mRNA expression correlates with HMGA2. Inverse correlation between PPARgamma mRNA expression and HMGA2 measured in hWAT of 134 individuals.
**Fig. 6****:**

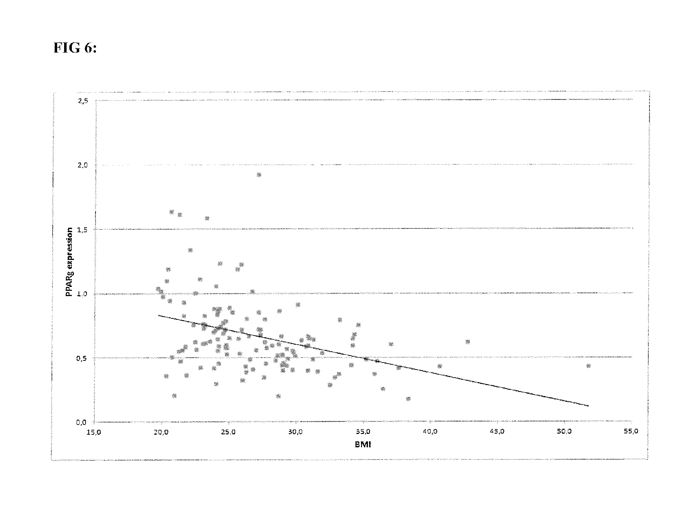

   Fig. 6: PPARgamma mRNA expression correlates with BMI. Inverse correlation between PPARgamma mRNA expression and BMI measured in hWAT of 134 individuals.
**Fig. 7****:**
   Fig. 7 is an exemplary diagram of a computing device comprising a client and/or server according to an implementation consistent with the principles of the invention.
**Fig. 8****:**
   Fig. 8 is a flowchart of exemplary processing of expression status for biomarker HMGA2 present in biological samples according to an implementation consistent with the principles of the present invention.

### EXAMPLES

Aspiration of subcutaneous white adipose tissue (WAT) is an easy, safe, and less invasive method to obtain adipose tissue. Herein, biomarkers and methods are described to identify these individuals based on gene expression analysis of transcripts expressed in samples taken from WAT. In particular but not restricted to it, it addresses the expression of mRNA of the gene encoding high mobility group protein AT-hook 2, p21, BAX, and Peroxisome proliferator-activated receptor gamma (PPARgamma). The high mobility group AT-hook 2 protein (HMGA2) apparently plays an important role of in adipocytic cell growth and development. When affected by chromosomal translocations its gene (HMGA2) is involved in the development of lipomas (Ashar et al., 1995; Schoenmakers et al., 1995). As to normal cells, later a couple of studies have demonstrated HMGA2's important role in adipocytic cell growth and development.

Transgenic mice expressing a truncated HMGA2 still retaining the three AT-hook domains present a giant phenotype with hyperplasia of WAT (Battista et al., 1999). Vice versa, HMGA2 knock-out mice not only show a pygmy phenotype with hypoplasia of WAT (Zhou et al., 1995) but also disruption of HMGA2 caused a reduction in the obesity induced by leptin deficiency in a gene-dose-dependent manner (Anand and Chada, 2000). Vernochet et al. (2002) have investigated the expression of the endogenous PPARγ gene during in vitro development of ES cells. Its pattern of expression was compared with that of developmental marker genes such as HMGA2. PPARgamma was found to be expressed early in embryoid bodies (EBs) derived from embryonic stem cells and in E.8.5 mouse embryos. Addition of a specific ligand for PPARgamma in developing EBs over-expressing PPARgamma did not commit stem cells towards the adipose lineage. In differentiated PPARgamma(-/-) EBs, only markers characteristic of preadipocytes were found to be expressed. Expression of the HMGA2 was weak in EBs and peaked early in differentiated embryonic outgrowths at a time when undifferentiated mesenchymal cells were proliferating. This suggests a critical PPARgamma-independent phase culminating in preadipocyte formation that precedes a PPARgamma-dependent phase in the development of adipose cells from pluripotent stem cells.

Thus, reduced levels of PPARgamma expression as well as elevated levels of HMGA2 expression can be assumed to reflect an increased level of immature fat cells. On the other hand HMGA2 is known to affect the p14Arf pathway implicating its role in modulating cellular senescence and apoptosis (Nishino et al., 2008; Markowski et al., 2011).

In the following investigations overweight was defined as a BMI >25.

Samples were taken either by fine needle aspiration or during abdominal surgery. After RNA isolation and cDNA synthesis the expression of the mRNAs of HMGA2, CDKN1A, p14Arf, BAX, and PPARgamma was determined by quantitative real time PCR.

### Example 1

It was shown that the expression of HMGA2 mRNA can be quantified in samples obtained by fine needle aspiration of subcutaneous adipose tissue.

### Fine needle aspiration

Aspirates were obtained from hWAT using a syringe with a disposable hypodermic needle (ø 0,9 mm). Immediately after aspiration 700 µl Qiazol Lysis Reagent (Invitrogen, Karlsruhe, Germany) was added.

### RNA Isolation

Total RNA was isolated using a RNeasy lipid tissue kit (Qiagen; Hilden, Germany) according to manufacturer's instructions and DNase I digestion was performed. Total RNA was isolated with Qiazol Lysis Reagent (Invitrogen) and purified on a Qiagen miRNeasy column (Qiagen). DNase digestion was carried out in the column during RNA extraction (RNase-free DNase Set, Qiagen).

### cDNA-Synthesis

About 250 ng of total RNA were reverse transcribed with 200 U/µl of M-MLV reverse transcriptase (Invitrogen), RNase Out and 150 ng random hexamers according to the manufacturer's instructions. RNA was denatured at 65°C for 5 min and subsequently kept on ice for 1 min. After adding the enzyme to the RNA primer mixes, samples were incubated for 10 min at 25°C to allow annealing of the random hexamers. Reverse transcription was performed at 37°C for 50 min followed by inactivation of the reverse transcriptase at 70°C for 15 min.

### Quantitative Real-Time PCR

Relative quantification of transcription levels was carried out by real-time PCR analyses using the Applied Biosystems 7300 Real-Time PCR system (Applied Biosystems, Darmstadt, Germany). A commercially available gene expression assay (Applied Biosystems) was used for quantification of HMGA2 (Hs00171569). HPRT served as endogenous control as described before (Markowski et al., 2010). All qRT-PCR experiments were done in triplicate.

### Results

Relative HMGA2 expression were measured in three samples (Fig. 1). The results clearly show that HMGA2 mRNA can be quantified even from small amount of adipose tissue cells as obtained by fine needle aspiration. Also, the amount of RNA isolated is sufficient to determine the expression of more than two genes (data not shown).

### Example 2

The expression of HMGA2 mRNA is highly elevated in samples of diabetic individuals compared to non-diabetic individuals.

### Tissue Samples

Samples of human subcutaneous abdominal adipose white tissue (hWAT) were taken during surgery and transferred into sterile Hank's solution. Prior to surgery, informed consent was obtained from all patients.

### RNA-Isolation

Total RNA was isolated using a RNeasy mini kit (Qiagen, Hilden, Germany) in a QIACube (Qiagen, Hilden, Germany) according to manufacturer's instructions and DNase I digestion was performed.

### cDNA-Synthesis

About 250 ng of total RNA were reverse transcribed with 200 U/µl of M-MLV reverse transcriptase (Invitrogen, Karlsruhe, Germany), RNase Out, 150 ng random hexamers and 10 mM dNTPs according to the manufacturer's instructions. RNA was denatured at 65°C for 5 min and subsequently kept on ice for 1 min. After adding the enzyme to the RNA primer mixes, samples were incubated for 10 min at 25°C to allow annealing of the random hexamers. Reverse transcription was performed at 37°C for 50 min followed by inactivation of the reverse transcriptase at 70°C for 15 min.

### Quantitative real-time PCR

Relative quantification of transcription levels was carried out by real-time PCR analyses using the Applied Biosystems 7300 real-time PCR system (Applied Biosystems, Darmstadt, Germany). For quantification of human HMGA2 (Hs00171569_m1 a commercially available gene expression assay (Applied Biosystems) was used. HPRT served as endogenous control as described before (Markowski et al., 2010).

### Statistical Analyses

The statistical significance of differences was assessed by the student's t test. In all comparisons, p < 0.05 was considered being statistically significant and p < 0.001 was considered being highly significant.

### Results

Statistically high significant (p < 0.001) differences of the level of HMGA2 mRNA were detected when comparing samples obtained from diabetic individuals (n = 11) vs. overweight individuals without diabetes (n = 68), diabetic individuals vs. non-overweight, non-diabetic individuals (n = 56), or diabetic individuals vs. non-diabetic individuals (n = 124) (Fig.1). Surprisingly, a comparison of the HMGA2 mRNA levels between overweight, non-diabetic individuals and non-overweight, non-diabetic individuals did not reveal statistically significant differences.

On the other hand, contrary to the expectations no correlation was noted between the expression of HMGA2 mRNA and the body mass index (BMI) (Fig.2).

### Example 3

Significant correlations were found between the expression of HMGA2 and p14Arf as well as CDKN1A. In contrast a significant inverse correlation between the expression of HMGA2 and PPARgamma was noted.

### Tissue Samples

Samples of human subcutaneous abdominal adipose white tissue (hWAT) were taken during surgery and transferred into sterile Hank's solution. Prior to surgery, informed consent was obtained from all patients.

### RNA-Isolation

Total RNA was isolated using a RNeasy mini kit (Qiagen, Hilden, Germany) in a QIACube (Qiagen, Hilden, Germany) according to manufacturer's instructions and DNase I digestion was performed.

### cDNA-Synthesis

About 250 ng of total RNA were reverse transcribed with 200 U/µl of M-MLV reverse transcriptase (Invitrogen, Karlsruhe, Germany), RNase Out, 150 ng random hexamers and 10 mM dNTPs according to the manufacturer's instructions. RNA was denatured at 65°C for 5 min and subsequently kept on ice for 1 min. After adding the enzyme to the RNA primer mixes, samples were incubated for 10 min at 25°C to allow annealing of the random hexamers. Reverse transcription was performed at 37°C for 50 min followed by inactivation of the reverse transcriptase at 70°C for 15 min.

### Quantitative real-time PCR

Relative quantification of transcription levels was carried out by real-time PCR analyses using the Applied Biosystems 7300 real-time PCR system (Applied Biosystems, Darmstadt, Germany). For quantification of human HMGA2 (Hs00171569_m1), CDKN1A (Hs99999142_m1), p14Arf (Hs00924091_m1), BAX (Hs00180269_m1), and PPARγ (Hs01115513_m1) commercially available gene expression assays (Applied Biosystems) were used. HPRT served as endogenous control as described before (Markowski et al., 2010).

### Statistical Analyses

The statistical significance of differences was assessed by the student's t test. In all comparisons, p < 0.05 was considered being statistically significant.

### Results

Significant correlations were noted between the expression of p14Arf and HMGA2 (Fig. 1) as well as between the expression of CDKN1a (p21) and HMGA2 (Fig. 2).

No significant correlation was noted between the expression of HMGA2 and BAX (data not shown). On the other hand a highly significant correlation was noted with the expression of CDKN1A and BAX (Fig. 3). The data also revealed a significant inverse correlation between the BMI and the expression of PPARgamma mRNA (Fig. 4).

## Claims

1. A method for diagnosis and/or prognosis of diabetes, comprising the steps of
a) analyzing in a sample of a subject the expression level or status of the gene HMGA2 wherein,
b) if HMGA2 shows an elevated expression equal to or above a cut-off value when compared to a control,
c) the subject is classified as a subject with diabetes or the subject is classified as a subject with poor prognosis or the subject is classified as a subject being at an elevated risk of becoming diabetic.

2. Method according to claim 1, wherein the diabetes is a type 2 diabetes.

3. Method according to claims 1 or 2, wherein the subject is a human and said human is adipose.

4. Method according to any of the preceding claims, wherein the control is selected from the group from the group of housekeeping genes as e.g. *HPRT, 18S, GAPDH, GUSB, PBGD, B2M, ABL, RPLP0.*

5. Method according to any of the preceding claims, wherein elevated expression is defined as the cut-off value being 1.5fold elevated when compared to the control or wherein the cut-off value is determined by a ROC (receiver operation curve) to adopt the cut-off value to national or geographic cohorts.

6. Method according to any of the preceding claims, wherein the sample is white adipose tissue (WAT).

7. Method according to any of the preceding claims, wherein the expression level is determined by quantitative reverse transcriptase PCR (qRT-PCR) as e.g. a Real-Time PCR reaction or by other possible methods include array hybridization, mass spectrometry (MALDI-TOF), serial analysis of gene expression (SAGE) and other sensitive method to detect mRNAs or proteins or fragments thereof.

8. Use of a nucleic acid molecule that hybridizes under stringent conditions to the mRNA transcript of the HMGA2 gene for determining the expression level of said gene in a method according to any of the above claims.

9. Use according to claim 8, wherein the nucleic acid is 15 to 100 nt in length.

10. Use according to claim 8 or 9, wherein the nucleic acid is a primer or probe and optionally the primer or probe is labelled.

11. Use of a kit for the diagnosis of diabetes comprising a nucleic acid that hybridizes under stringent conditions to the mRNA transcript of the HMGA2 gene.

12. Use according to claim 11, wherein the kit is Real-Time PCR kit.
